# EUROPEAN PATENT APPLICATION

(11) **EP 2 733 211 A2**
(43) Date of publication of application: **21.05.2014**
(21) Application number: 12791214.5
(22) Date of filing: 26.06.2012
(51) Int. Cl.: C12N 15/82

(54) **METHOD FOR INCREASING OR DECREASING THE DEVELOPMENT OF SYLLEPTIC OR PROLEPTIC BRANCHING IN A LIGNEOUS PLANT**

(30) Priority: 13.07.2011 ES 201131186 P
(71) Applicant: Universidad Politécnica De Madrid, 28040 Madrid (ES)
(72) Inventor: ALLONA ALBERICH, Isabel, E-28233 Pozuelo de Alarcón - Madrid (ES); MORENO CORTÉS, Alicia, E-28233 Pozuelo de Alarcón - Madrid (ES); ARAGONCILLO BALLESTEROS, Cipriano, E-28233 Pozuelo de Alarcón - Madrid (ES)
(74) Representative: ABG Patentes, S.L.
(86) International application number: PCT/ES2012/070471
(87) International publication number: WO 2013/007848

(57) **Abstract**

The present invention consists of a biotechnological application of the *RAV1* gene (related to ABI3 and Viviparous 1) and its homologue *RAV2* in relation to their capacity to increase or reduce the development of sylleptic and/or proleptic branches in ligneous species when their expression levels or the activity of the proteins they encode are modified. It is possible to increase the biomass production of a ligneous species plantation or to reduce the number of nodes in the trunk of ligneous species of logging interest by means of modifying the expression of said genes.

## Description

### Technical Field

The invention is encompassed in the technical field of biotechnology and forestry practice for energy, chemistry or logging purposes.

### Background of the Invention

Short rotation forestry (SRF) of fast growing forestry species under an intensive management system is one of the most interesting sources of biomass. The main species exploited with this model are poplar (*Populus spp*.), eucalyptus (*Eucalyptus spp.*) or willow (*Salix spp.*) with a turn of logging between 2 and 10 years. On one hand, the lignocellulosic biomass is suitable for producing biofuels in the form of liquid fuels or gases for transport, and bioliquids or combustibles liquids intended for energy uses other than transport, such as for producing electricity or heat. On the other hand, such forestry plantations can be established in surplus marginal or agricultural lands, therefore they do not compete directly with the food crop for fertile soils. However, the yield thereof for bioenergetics purposes is still subject to improvement since it remains as an important limiting factor which is far from being economical and commercially viable.

Lignocellulosic biomass yield is a highly complex genetic trait. It is the integrated and combined result of many other complex traits, each of them is in turn controlled by several genes. These traits include, for example, the height of the tree, the diameter of the trunk, the number and area of the leaves or the number of branches. In recent years, some research equipment have dealt with the problem through identifying the genomic regions involved in the regulation of these traits in ligneous species by means of QTL (*Quantitative Trait Loci*) mapping. In this sense, the work of Rae et al. (Five QTL hotspots for yield in short rotation coppice bioenergy poplar: the Poplar Biomass Loci. BMC Plant Biology 2009, vol. 9, p. 23) conducted with a family of 320 genotypes of the *Populus trichocarpa* x *P. deltoides* hybrid cultivated in short rotations is especially interesting. Five QTLs responsible for 20% of the variation among these genotypes in terms of biomass yield are identified in this work. It further describes the positive correlations existing between diameter and basal area of stems and branches from the first year and their biomass yield in that year and in the next five years, and between the number of sylleptic branches growing in the first year and the basal diameter and area of stems and branches from the first year. In most ligneous species in temperate latitudes, the lateral buds do not sprout during the same season in which they are formed. These buds, proleptic, must survive the winter to be able to sprout and form branches the next spring. In contrast, sylleptic buds which appear particularly during the juvenile stage of the tree do not need cold winters to develop into branches.

Therefore, sylleptic branching does not only significantly increase the general growth of poplar, particularly throughout the first tears, but it also presents itself as a valuable trait which allows an early prediction the final biomass yield of a plantation.

The *RAV1* gene (Related to ABI3 and Viviparous 1) belongs to the family of transcription factors RAV, characterized by the presence of the DNA-binding domains AP2/EREBP (Apetala2/Ethylene Response Element Binding Factor) and B3 in its primary structure (Kagaya et al., RAV1, a novel DNA-binding protein, binds to bipartite recognition sequence through two distinct DNA-binding domains uniquely found in higher plants. Nucleic acids research 1999, vol. 27, p. 470-478; Yamasaki et al., Solution structure of the B3 DNA binding domain of the Arabidopsis cold-responsive transcription factor RAV1. The plant cell 2004, vol. 16, p. 3448-3459) According to the literature, in herbaceous plant *Arabidopsis thaliana* the genes *RAV1* (*AtRAV1;* AT1G13260) and *RAV2* (*AtRAV2*/*TEM2;* AT1G68840) would be involved in abscisic acid-independent cold response (Sakuma et al., DNA-binding specificity of the ERF/AP2 domain of Arabidopsis DREBs, transcription factors involved in dehydration- and cold-inducible gene expression. Biochemical and biophysical research communications 2002, vol. 290, p. 998-1009) and in the response to other external stimuli such as mechanical stimuli (Kagaya and Hattori, Arabidopsis transcription factors, RAV1 and RAV2, are regulated by touch-related stimuli in a dose-dependent and biphasic manner. Genes and genetic systems 2009, vol. 84, p. 95-99). *AtRAV1* may also act as a negative regulator in the development of lateral roots and rosette leaves and together with the genes *TEMPRANILLO1* (*TEM1;* AT1G25560) and *AtRAV2*/*TEM2,* in flowering time (Hu et al., Arabidopsis RAV1 is down-regulated by brassinosteroid and may act as a negative regulator during plant development. Cell research 2004, vol. 61, p. 3947-3947; Castillejo and Pelaz, The balance between CONSTANS and TEMPRANILLO activities determines FT expression to trigger flowering. Current biology 2008, vol. 18, p. 1338-1343). *AtRAV1* has also been described as a positive regulator in leaf senescence process (Woo et al., The RAV1 transcription factor positively regulates leaf senescence in Arabidopsis. Journal of experimental botany 2010, vol. 61, p. 3947-3957). On the other hand, in tomato (*Solanum lycopersicum*), the *RAV2* gene modulates the defense response against the pathogenic bacteria *Ralstonia solanacearum* (Li et al., Tomato RAV transcription factor is a pivotal modulator involved in the AP2/EREBP-mediated defense pathway. Plant Physiology 2011, vol. 156, p. 213-227). In these works, the function of A. *thaliana* genes *AtRAV1* (Hu et *al.,* 2004), *TEM1* and *AtRAV2*/*TEM2* (Castillejo and Pelaz, 2008) has been related with the control of the aspects of growth and the development different from branching regulation, since manipulating their expression in this herbaceous species does not cause a higher number of branches. However, none of these prior art publications relates the RAV1 gene with the regulation of branching development in any ligneous species.

Application WO 2006/132616 A1 describes the regulatory function of genes *RAV1 and RAV2* in plant gene silencing. This patent has a generic interest in plant genetic manipulation technology, but its protected subject-matter does not relate to the development processes controlled by said RAV genes nor does it relate to plant productivity.

On the other hand, the suppression of genes *RAV1* and *RAV2* expression should inhibit the appearance of branches and lead to less nodes in the tree trunk, whereby high quality woods would be obtained. The inhibition of sylleptic and/or proleptic branching is thus revealed as a possible tool with great commercial value.

Therefore, the problem that arises in the art effectively is to get a modification in the amount or quality of wood production from a ligneous plant, as needed, to increase the profit of its derivative products. The solution proposed by the present invention is to modify the expression of genes *RAV1* and *RAV2* in said ligneous plant and thus influence its sylleptic and/or proleptic branching production by technical methods.

### Description of the Invention

The present invention describes a gene modification applicable to ligneous species which allows modifying the development of sylleptic branches since the plants are very young.

It consists of a biotechnological application of the *RAV1* gene (Related to ABI3 and Viviparous 1) and its homologue *RAV2* in relation to their capacity for increasing or reducing the degree of development of sylleptic and/or proleptic branching in a ligneous species when their expression levels, or the activity of the proteins encoded by them are modified.

Therefore, a first embodiment of the invention is a method for increasing or reducing the development of sylleptic and/or proleptic branching in a ligneous plant with respect to its wild variety, which comprises modifying the expression of genes *RAV1* and/or *RAV2,* or of their functional derivatives. This expression modification can be performed in the whole plant or specifically in a tissue or organ and can be permanent or temporary.

In the scope of the present application, "expression of a gene" is understood as the protein product resulting from the array of mechanisms decoding the information contained in said gene processed by means of transcription, translation and post-translational modifications to the final form of the protein.

The inventors have observed that, upon expressing the coding region of the *RAV1* gene isolated from *Castanea sativa* Miller (*CsRAV1*) under the control of a constitutive promoter in the *Populus tremula x P. alba* (clone INRA 717 1-B4) hybrid, all the individuals of the ten transformation events which have been analyzed develop sylleptic branches from the lateral buds in a short time period. This time period is approximately one month after their transplant to soil from *in vitro* cultivation. In contrast, the development described does not occur in the wild type (WT) untransformed control poplars. It is deduced from this observation that the continuous overexpression of the CsRAV1 gene is responsible for the early formation of sylleptic branches in poplar.

On the other hand, given the high sequence identity existing between the proteins RAV1 and RAV2 of *Populus trichocarpa* (91.9%) and therefore, their possible functional redundancy, the increase in the expression of a *RAV2* gene may have a similar effect on branching as the overexpression of *CsRAV1.*

The genome of the genus *Populus* and specifically of the species *P*. *trichocarpa,* the sequence of which is known and can be accessed freely through the NCBI or Phytozome public database, contains five genes encoding RAV transcription factors, two of which known as *PtRAV1* and *PtRAV2* are homologous to the genes *AtRAV1, AtRAV2*/*TEM2* and *TEM1* mentioned above. Other ligneous species such as *Eucalyptus grandis, Vitis vinífera, Citrus clementina, C. sinensis* or *Prunus persica,* the genomic sequences of which are available in public databases, also contain genes homologous to *PtRAV1, PtRAV2, AtRAV1, AtRAV2*/*TEM2* and *TEM1.*

These are examples of polynucleotides originating from other plant species sharing a common evolutionary origin with *CsRAV1,* i.e., polynucleotides homologous to *CsRAV1* encoding proteins with completely or partially equal or equivalent functions and which could also be used entirely or partially in the method of the invention. Therefore, a new aspect of the invention relates to the nucleotide sequences of other genes encoding polypeptides which, like CsRAV1, simultaneously contain the AP2 and B3 domains (Kagaya et al., RAV1, a novel DNA-binding protein, binds to bipartite recognition sequence through two distinct DNA-binding domains uniquely found in higher plants. Nucleic acids research 1999, vol. 27, p. 470-478). The AP2 and B3 domains will be identified by means of bioinformatics applications such as Basic Local Alignment Search Tool (BLAST; Altschul et al., Gapped BLAST and PSI-BLAST: a new generation of protein database search programs. Nucleic Acid Research 1997, vol. 25, p. 3389-3402) or Conserved Domain Database (CCD; Marchler-Bauer et al., CDD: a Conserved Domain Database for the functional annotation of proteins. Nucleic Acid Research 2011, vol. 39, p. D225-D229).
The first aspect of the invention is a tool for increasing the biomass production of a forestry plantation genetically modified in this manner, the biotechnological application of which is of great interest in various industrial sectors.

Therefore, one embodiment is the method of the invention, wherein said modification of the expression of *RAV1* and/or *RAV2* is an overexpression. Said overexpression preferably comprises introducing a gene construct in the plant. In a more preferred embodiment, said gene construct comprises the fusion of a gene encoding a polypeptide simultaneously containing the AP2 and B3 domains, or their functional derivative to a promoter; said gene is preferably *RAV1* and/or *RAV2* and is more preferably *CsRAV1.* In another preferred embodiment, the promoter used is a constitutive promoter, preferably CaMV35S, cauliflower mosaic virus 35S, or its functional derivative. In another preferred embodiment, said promoter is an inducible promoter. Another embodiment complementary to the foregoing is that said promoter is a tissue- or organ-specific promoter.

Another embodiment of the invention is the *CsRAV1* gene isolated from *Castanea sativa* Miller which regulates the sylleptic and proleptic production of the species and is identified by SEQ ID NO: 1. This SEQ ID NO: 1 as is reported includes an untranslated 5' region prior to the start codon, such that a preferred embodiment is the *CsRAV1* identified by a sequence with at least 91.7% of identity with respect to SEQ ID NO: 1. Another preferred embodiment is the protein obtained in the expression of *CsRAV1* identified by SEQ ID NO: 2. The gene and the resulting protein can be used entirely or partially in the methods of the present invention.

Another preferred embodiment is an expression vector comprising the CsRAV1 gene and the promoter CaMV35S, or their functional derivatives. Another very preferred embodiment is a ligneous plant with at least one CsRAV1-encoding polynucleotide, or its functional analogs, stably integrated in the genome of the cells.

Another embodiment of the invention is the method of the invention wherein said overexpression is obtained at least by a mutation or an insertion of T-DNA in a gene encoding a polypeptide simultaneously containing the AP2 and B3 domains, said gene is preferably *RAV1* and/or *RAV2,* or their functional derivatives.

In contrast, a reduction in the expression of the *RAV1* gene in a ligneous species in which the sylleptic branching is frequent must result in the reduction thereof or in the reduction of proleptic branching the following year.

This second aspect of the present invention does not only include the individual reduction in the expression of the genes *RAV1* or *RAV2* but it can include the joint reduction in the expression of both genes. That is to say, it would reduce the sylleptic and/or proleptic branching in a ligneous species thank of the reduction in the expression of genes *RAV1* and/or *RAV2* by gene silencing techniques, through the endogenous production of antibodies specific for the proteins RAV1 and/or RAV2 or by means of introducing mutations or T-DNA insertions.

According to the foregoing, another embodiment is the method of the invention in which said modification of the expression of *RAV1* and/or *RAV2* is a reduction in expression.

This reduction in expression preferably comprises gene silencing, more preferably by interference RNAs, microRNAs or antisense messengers. Another embodiment is that said reduction comprises introducing a gene construct to the plant, and that preferably said gene construct comprises the fusion to a promoter of a gene encoding a polypeptide simultaneously containing the AP2 and B3 domains or their functional derivative. Said gene is preferably *RAV1* and/or *RAV2.* A more preferred embodiment is that said promoter is a constitutive promoter, preferably CaMV35S, cauliflower mosaic virus 35S, or its functional derivative. Another preferred embodiment is that said promoter is an inducible promoter. Another embodiment complementary to the foregoing is that the promoter is a tissue-or organ-specific promoter.

Another preferred embodiment of the invention is that the reduction in expression comprises the endogenous production of at least one antibody specific for a protein simultaneously containing the AP2 and B3 domains; said protein is preferably RAV1 and/or RAV2.

Another preferred embodiment is that the reduction in expression comprises at least one mutation or an insertion of T-DNA in a gene encoding a polypeptide simultaneously containing the AP2 and B3 domains; said gene is preferably RAV1 and/or RAV2 or their functional derivatives.

This second aspect of the invention involves a tool for reducing the number of nodes in the trunk in a ligneous species of logging interest genetically modified in this manner.

The techniques for obtaining transgenic constructs and plants, as well as for handling the bioinformatics analysis tools referred to in the present application belong to the scope of genetic engineering, *in vitro* cultivation and bioinformatics and they are widely known by the persons skilled in the art.

In one way or another, the genetic modification of the invention is potentially applicable to any genotype of a ligneous species, it therefore allows taking advantage of the adaptive characteristics of said genotype to a specific habitat.

The present invention is therefore applicable in various industrial sectors. The increase in the biomass yield of short rotation forestry plantations may be interesting to the energy industry interested in using biomass for biofuel production, the chemical industry interested in preparing chemical products from this biomass, as well as the silvicultural industries and paper industries.

Therefore, another additional embodiment of the invention is a cell of ligneous plant with modified RAV1 and/or RAV2 expression. Another preferred embodiment is a transgenic ligneous plant with modified RAV1 and/or RAV2 expression. Another embodiment is the plant product obtained from that transgenic ligneous plant, preferably a bioliquid and more preferably a biofuel.

On the other hand, the reduction in sylleptic and/or proleptic branching and, therefore in the number of nodes in the trunk of a ligneous species can also be interesting for silvicultural and logging industries interested in utilizing harvestable forestry.

Therefore, another very preferred embodiment is the wood obtained from a transgenic ligneous plant with modified RAV1 and/or RAV2 expression.

### Brief Description of the Drawings

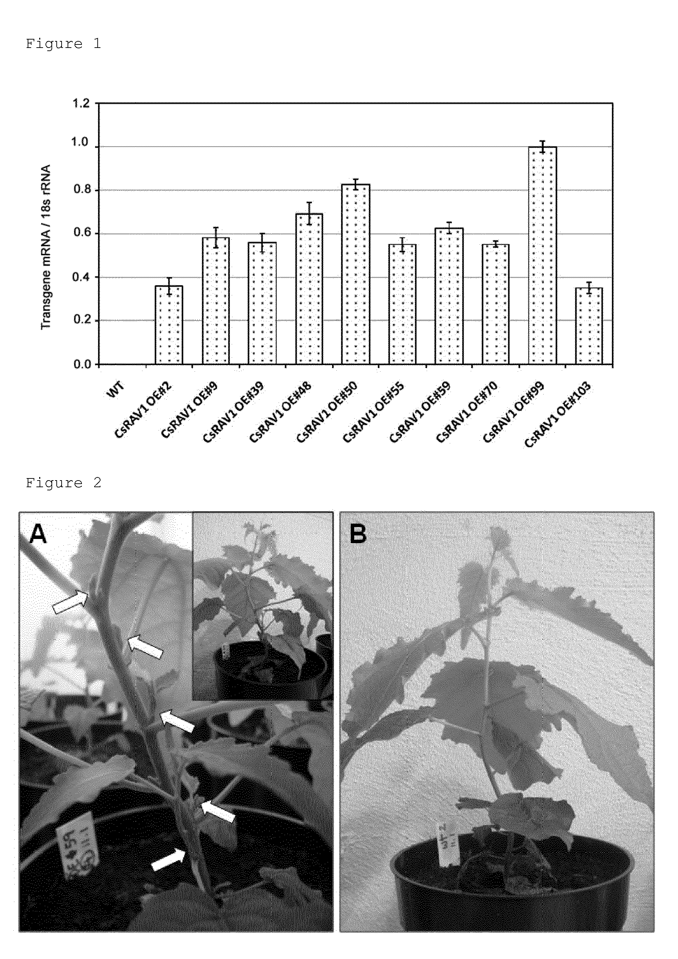

Figure 1:relative abundance of the *CsRAV1* transgene in ten independent transformants of the *Populus tremula* x *P. alba* (clone INRA 717 1-B4) hybrid. The independent transformants or events are designated as CsRAV1 OE (*overexpressor*) followed by the symbol # and a number. The values represent the average of three technical replicates ±SD (standard deviation).
Figure 2: images of (A) the CsRAV1 OE #59 event of Figure 1 of *Populus tremula* x *P. alba* (clone INRA 717 1-B4), where the development of sylleptic branches from the lateral buds shown with arrows accompanied by a general image of said event is seen; and (B) a wild type *Populus tremula* x *P. alba* (clone INRA 717 1-B4) individual in which this development does not occurs. The plants are 35-day old, counted from the transplant to soil from the *in vitro* cultivation.

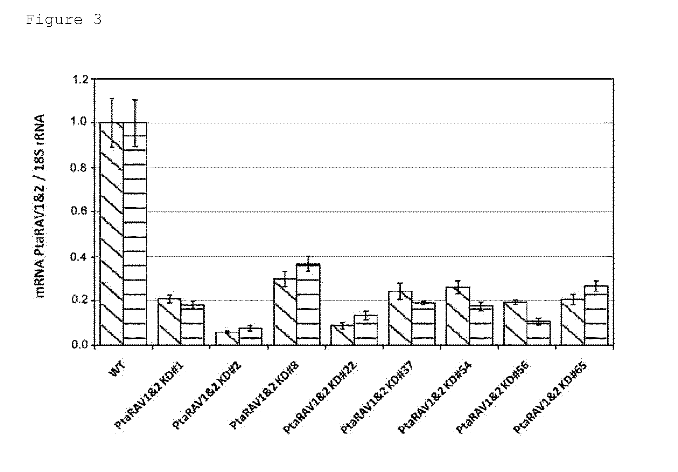

Figure 3: relative abundance of the genes *PtaRAV1* (bars with diagonal stripes) and *PtaRAV2* (bars with horizontal stripes) in eight independent transformants of the *Populus tremula* × *P. alba* (clone INRA 717 1-B4) hybrid. The independent transformants or events are designated as PtaRAV1&2 KD (*knockdown*) followed by the symbol # and a number. The values represent the average of three technical replicates ±SD (standard deviation).

### Examples

The following examples are provided to the present invention in an illustrative but non-limiting manner.

### Example 1: Overexpression of poplar RAV1 and RAV2

The coding region of the *RAV1* gene isolated from stems of *Castanea sativa Miller* (*CsRAV1*) was cloned in the binary vector pGWB2 (Nakagawa et al., Development of series of gateway binary vectors, pGWBs, for realizing efficient construct of fusion genes for plant transformation. Journal of bioscience and bioengineering 2007, vol. 104, p. 34-41) carrier of the constitutive promoter CaMV35S (*cauliflower mosaic virus* 35S). This construct was used for transforming the explants obtained from 4-week old young seedlings of the *Populus tremula* × *P. alba* (clone INRA 717 1-B4) hybrid cultivated *in vitro* via infection with *Agrobacterium tumefaciens* (strain GV3101/pMP90). In all the stages of the *in vitro* cultivation, the media were prepared with 1X Murashige & Skoog 1B (Duchefa), 2% sucrose (Merck), 0.7 or 0.8% agar (explants and calluses or shoots and seedlings, respectively; BD Bacto), and were adjusted to pH 5.8 with 0.1 N sodium hydroxide. After two days of cultivation in a medium supplemented with 0.01 mg/L thidiazuron (Sigma-Aldrich) and 1 mg/L 2,4-dichlorophenoxyacetic acid (Sigma-Aldrich) to induce cell dedifferentiation in these explants, they were infected by submerging them for 15 minutes in a 2YT *A. tumefaciens* culture (16 g/L BD Bacto tryptone, 10 g/L BD Bacto yeast extract, 5 g/L sodium chloride Merck) with an optical density of 0.05 (λ=660). The explants thus infected continued to be cultivated in the same medium for two more days. They were then transferred to a medium supplemented with 0.02 mg/L thidiazuron, 1 mg/L 2,4-dichlorophenoxyacetic acid, 50 mg/L kanamycin sulfate (Roche), 20 mg/L hygromycin B (Duchefa) and 250 mg/L cefotaxime (Duchefa), and cultivated until obtaining calluses of 0.5 cm³ in size. These calluses were then transferred to a medium supplemented with 0.004 mg/L thidiazuron, 0.05 mg/L naphthaleneacetic acid (Sigma-Aldrich), 50 mg/L kanamycin sulfate, 20 mg/L hygromycin B and 250 mg/L cefotaxime so that their cells started to differentiate again and developed into shoots. Finally, when these shoots reached a height of 1 cm, they were dissected and rooted in a medium supplemented with 0.5 mg/L indolaecetic acid (Sigma-Aldrich), 50 mg/L kanamycin sulfate, 20 mg/L hygromycin B and 125 mg/L cefotaxime for generating whole poplar seedlings. Once analyzed, the seedlings expressing the transgene (Figure 1) were selected, transplanted to soil and cultivated in a greenhouse under long day conditions, 16 hours of light for 8 hours of darkness, at a temperature of 20±2°C. In these conditions and in the period between 30 and 40 days, when the plants reached a height between 30 and 40 cm, they started to develop sylleptic branching. In contrast, the non-transgenic control plants did not do so (Figure 2).

### Example 2: Analysis of transgene expression by qRT-PCR

Before transferring them to ground, the seedlings of Example 1 were analyzed by means of quantitative real-time PCR (qRT-PCR) to detect and quantify the expression of the transgene integrated in their nuclear genome and to dismiss those events in which the transgene was not expressed. The methods used for extracting total RNA for cDNA synthesis, as well as the composition and the conditions in which the qRT-PCR reactions were performed are detailed by Ibañez et al., Overall alteration of circadian clock gene expression in the chestnut cold response. PLoS ONE 2008, vol. 3, e3567 (doi: 10.1371/journal.pone.0003567). The RNA used for carrying out said analysis was obtained from 2-month old young plant leaves cultivated *in vitro.* The primers used for detecting the expression of *CsRAV1* were those identified by SEQ ID NO: 3 and SEQ ID NO: 4. The analysis of the relative abundance of the transgene *CsRAV1* in wild type individuals represents the negative control. 18s ribosomal RNA was used as a reference using the primers identified by SEQ ID NO: 5 and SEQ ID NO: 6 described in Böhlenius et al. (CO/FT regulatory module controls timing of flowering and seasonal growth cessation in trees. Science 2006, vol. 312, p. 1040-1043).

### Example 3: Reduction in the expression of poplar RAV1 and/or RAV2

In order to reduce the expression of the genes *RAV1* and *RAV2,* a hairpin interfering RNA (*hpiRNA*) construct from the *RAV1* sequence of *Populus* alba -homologue to CsRAV1 gene- was generated in the binary vector pHELLSGATE12 (Helliwell and Waterhouse, Constructs and methods for high-throughput gene silencing in plants. Methods 2003, vol. 30, p. 289-295), carrier of the constitutive promoter CaMV35S. Specifically, to make this construct the region comprised between the DNA-binding domains AP2/EREBP and B3 of the *RAV1* gene of *P*. *alba,* the sequence of which is specified in SEQ ID NO: 7, was used Given the high overall degree of homology existing between the DNA coding sequences of the genes *RAV1* (NCBI Reference Sequence XM_002315922.1) and *RAV2* (XM_002311402.1) of *P*. *trichocarpa* (91.1%), the construct was designed for silencing the joint expression of the endogenous genes *RAV1* and *RAV2* and thus preventing a possible partial effect on branch development inhibition. This construct was used for transforming explants obtained from 4-week old young seedlings of the *Populus tremula* x *P. alba* (clone INRA 717 1-B4) hybrid cultivated *in vitro* through infection with *Agrobacterium tumefaciens* (cepa GV3101/pMP90). In all the stages of the *in vitro* cultivation, the media were prepared with 1X Murashige & Skoog 1B (Duchefa), 2% sucrose (Merck), 0.7 or 0.8% agar (explants and calluses or shoots and seedlings, respectively; BD Bacto), and were adjusted to pH 5.8 with 0.1 N sodium hydroxide. After two days of cultivation in a medium supplemented with 0.01 mg/L thidiazuron (Sigma-Aldrich) and 1 mg/L 2,4-dichlorophenoxyacetic acid (Sigma-Aldrich) to induce cell dedifferentiation in these explants, they were infected by submerging them for 15 minutes in a 2YT *A. tumefaciens* culture (16 g/L BD Bacto tryptone, 10 g/L BD Bacto yeast extract, 5 g/L sodium chloride Merck) with an optical density of 0.05 (λ=660). The explants thus infected continued to be cultivated in the same medium for two more days. They were then transferred to a medium supplemented with 0.02 mg/L thidiazuron, 1 mg/L 2,4-dichlorophenoxyacetic acid, 50 mg/L kanamycin sulfate (Roche), 20 mg/L hygromycin B (Duchefa) and 250 mg/L cefotaxime (Duchefa), and cultivated until obtaining calluses of 0.5 cm³ in size. These calluses were then transferred to a medium supplemented with 0.004 mg/L thidiazuron, 0.05 mg/L naphthaleneacetic acid (Sigma-Aldrich), 50 mg/L kanamycin sulfate, 20 mg/L hygromycin B and 250 mg/L cefotaxime so that their cells started to differentiate again and develop into shoots. Finally, when these shoots reached a height of 1 cm, they were dissected and rooted in a medium supplemented with 0.5 mg/L indolaecetic acid (Sigma-Aldrich), 50 mg/L kanamycin sulfate, 20 mg/L hygromycin B and 125 mg/L cefotaxime for generating whole poplar seedlings. Once analyzed, the seedlings in which the expression of the endogenous genes *PtaRAV1* and *PtaRAV2* had been diminished with respect to the expression of said genes in wild type seedlings (Figure 3) were selected. These seedlings transplanted to soil and cultivated in a greenhouse under long day conditions, 16 hours of light for 8 hours of darkness, at a temperature of 20±2°C. The results of the proleptic branching can be obtained after the winter of 2011-2012. The logical hypothesis is that these plants must develop a lower number of branches and, therefore, the number of nodes in the wood obtained from their stems will also be less, thus increasing the commercial value of the wood.

### Example 4: Analysis of PtaRAV1 and PtaRAV2 expression by qRT-PCR

Before transferring them to soil, the seedlings obtained from Example 3 were analyzed by means of quantitative real-time PCR (qRT-PCR) to quantify the expression of the endogenous genes *PtaRAV1* and *PtaRAV2* and to select those in which the expression thereof had been diminished with respect to their expression in wild type seedlings. The methods used for extracting total RNA for cDNA synthesis, as well as the composition and the conditions in which the qRT-PCR reactions were performed are detailed by Ibañez et al., Overall alteration of circadian clock gene expression in the chestnut cold response. PLoS ONE 2008, vol. 3, e3567 (doi: 10.1371/journal.pone.0003567). The RNA used for carrying out said analysis was obtained from 2-month old young plant leaves cultivated *in vitro* subjected to a temperature of 4°C for 3 hours. The primers used for detecting the expression of *PtaRAV1* were those identified with SEQ ID NO: 8 and SEQ ID NO: 9; for *PtaRAV2* the primers were those identified with SEQ ID NO: 10 and SEQ ID NO: 11. The analysis of the relative abundance of the genes *PtaRAV1* and *PtaRAV2* in wild type individuals represents the positive control and the calibrator. 18s ribosomal RNA was used as a reference using the primers identified by SEQ ID NO: 5 and SEQ ID NO: 6 described in Böhlenius et al. (CO/FT regulatory module controls timing of flowering and seasonal growth cessation in trees. Science 2006, vol. 312, p. 1040-1043).

## Claims

1. Method for increasing or reducing the development of sylleptic and/or proleptic branching in a ligneous plant with respect to its wild variety which comprises modifying the expression of genes *RAV1* and/or *RAV2,* or of their functional derivatives.

2. A method according to claim 1, wherein said modification is performed in the whole plant.

3. A method according to claim 1, wherein said modification is performed specifically in a tissue or organ.

4. A method according to any of claims 1 to 3, wherein said modification is permanent.

5. A method according to any of claims 1 to 3, wherein said modification is temporary.

6. A method according to any of claims 1 to 5, wherein said modification of the expression of *RAV1* and/or *RAV2* is an overexpression.

7. A method according to claim 6, wherein said overexpression comprises introducing a gene construct in the plant.

8. A method according to claim 7, wherein said gene construct comprises the fusion to a promoter of a gene encoding a polypeptide simultaneously containing the AP2 and B3 domains, or their functional derivative.

9. A method according to claim 8, wherein said gene is *RAV1* and/or *RAV2.*

10. A method according to claim 9, wherein said gene is the gene *CsRAV1* from *Castanea sativa* Miller identified by SEQ ID NO: 1.

11. A method according to any of claims 8 to 10, wherein said promoter is a constitutive promoter.

12. A method according to claim 11, wherein said constitutive promoter is CaMV35S, cauliflower mosaic virus 35S, or its functional derivative.

13. A method according to any of claims 8 to 10, wherein said promoter is an inducible promoter.

14. A method according to any of claims 8 to 13, wherein said promoter is a tissue- or organ-specific promoter.

15. The *CsRAV1* gene isolated from *Castanea sativa* Miller identified by SEQ ID NO: 1.

16. *CsRAV1* isolated from *Castanea sativa* Miller identified by a sequence with at least 91.7% of identity with respect to SEQ ID NO: 1.

17. The protein obtained in the expression of *CsRAV1* identified by SEQ ID NO: 2.

18. Expression vector comprising the gene CsRAV1 and the promoter CaMV35S, or their functional derivatives.

19. Ligneous plant with at least one CsRAV1-encoding polynucleotide, or its functional analogs, stably integrated in the genome of the cells.

20. A method according to claim 6, wherein said overexpression is obtained at least by a mutation or an insertion of T-DNA in a gene encoding a polypeptide simultaneously containing the AP2 and B3 domains.

21. A method according to claim 20, wherein said gene is *RAV1* and/or *RAV2,* or their functional derivatives.

22. A method according to any of claims 1 to 5, wherein said modification of the expression of *RAV1* and/or *RAV2* is a reduction in expression.

23. A method according to claim 22, wherein said reduction in expression comprises gene silencing.

24. A method according to claim 23, wherein said gene silencing is obtained by interference RNAs, microRNAs or antisense messengers.

25. A method according to any of claims 22 to 24, wherein said reduction comprises introducing a gene construct to the plant.

26. A method according to claim 25, wherein said gene construct comprises the fusion to a promoter of a gene encoding a polypeptide simultaneously containing the AP2 and B3 domains, or their functional derivative to a promoter.

27. A method according to claim 26, wherein said gene is *RAV1* and/or *RAV2.*

28. A method according to one of claims 26 or 27, wherein said promoter is a constitutive promoter.

29. A method according to claim 28, wherein said constitutive promoter is CaMV35S, cauliflower mosaic virus 35S, or its functional derivative.

30. A method according to one of claims 26 or 27, wherein said promoter is an inducible promoter.

31. A method according to any of claims 26 to 30, wherein said promoter is a tissue- or organ-specific promoter.

32. A method according to claim 22, wherein said reduction comprises the endogenous production of at least one specific antibody against a protein simultaneously containing the AP2 and B3 domains.

33. A method according to claim 32, wherein said protein is RAV1 and/or RAV2.

34. A method according to claim 22, wherein said reduction is obtained at least by a mutation or an insertion of T-DNA in a gene encoding a polypeptide simultaneously containing the AP2 and B3 domains.

35. A method according to claim 34, wherein said gene is *RAV1* and/or *RAV2,* or their functional derivatives.

36. Cell of ligneous plant with modified RAV1 and/or RAV2 expression.

37. Transgenic ligneous plant with modified RAV1 and/or RAV2 expression.

38. Plant product obtained from a transgenic ligneous plant with modified RAV1 and/or RAV2 expression.

39. A plant product according to claim 38, wherein said plant product is a bioliquid.

40. A plant product according to claim 39, wherein said bioliquid is a biofuel.

41. Wood obtained from a transgenic ligneous plant with modified RAV1 and/or RAV2 expression.
